# EUROPEAN PATENT APPLICATION

(11) **EP 2 444 794 A1**
(43) Date of publication of application: **25.04.2012**
(21) Application number: 10789540.1
(22) Date of filing: 16.06.2010
(51) Int. Cl.: G01N 21/77, G01N 33/579

(54) **OPTICAL REACTION MEASUREMENT DEVICE AND OPTICAL REACTION MEASUREMENT METHOD**

(30) Priority: 19.06.2009 JP 2009146821
(71) Applicant: Kowa Company, Ltd., Nagoya-shi, Aichi-ken 460-8625 (JP)
(72) Inventor: HARA, Takuya, Hamamatsu-shi Shizuoka 431-2103 (JP); KATO, Yoichi, Hamamatsu-shi Shizuoka 431-2103 (JP)
(74) Representative: Elsy, David
(86) International application number: PCT/JP2010/060249
(87) International publication number: WO 2010/147166

(57) **Abstract**

Provided is an optical reaction measurement device that measures whether a reaction is occurring between a sample and a reagent, or how a reaction is progressing, on the basis of changes over time of optical properties of a liquid mixture of the sample and the reagent. The provided technique makes it possible to reduce variation in the relationship between the time at which the sample and the reagent are mixed and the time at which measurement of optical properties begins, thereby improving measurement precision. The preparation time required for tasks such as mixing the sample and the reagent is predetermined, and when a start switch on the measurement device is turned to "on," a timer starts running, and the time remaining until the preparation time begins is shown to an operator as a countdown. When the count reaches zero, the operator is informed that the preparation time has begun. The amount of time that has passed since the preparation time began is used as time data to accompany measurement data, and optical property data from after the end of the preparation time is used in analysis.

## Description

### TECHNICAL FIELD

The present invention relates to an optical reaction measurement device and an optical reaction measurement method, which make use of the fact that the optical properties of a sample are modified by reacting to the addition of a given reagent to the sample, so as to measure the presence or absence, or the extent of progression, of the reaction between the sample and the reagent by means of an optical technique.

### BACKGROUND ART

The technology for causing the optical properties of a sample to be modified by a reaction between the sample and a reagent and then measuring the presence or absence, or the extent of progression, of the reaction between the sample and the reagent by acquiring the modification of the optical properties is well known. An example thereof includes the technology for measuring the concentration of water pollution components such as nitrogen, phosphorus, nitric acid, nitrous acid, ammonia, phosphoric acid, and heavy metals by using a specialized coloring agent that has been prepared in advance in order to measure the absorbance while a sample and a reagent are mixed in a measurement container and colorized. Another example thereof is an immunoassay in which a specific antigen-antibody reaction is caused by adding an antibody for an antigen or an antigen for an antibody as a reagent to react with the antigen or antibody in a specimen in order to optically detect aggregates formed by this antigen-antibody reaction.

Still another example thereof also includes the technology for optically measuring the gelation of the liquid mixture formed by the reaction in which an LAL reagent is added to a sample that contains a physiologically active substance of biological origins such as endotoxins and β-D-glucans. An additional description is provided below for this example.

Endotoxins are lipopolysaccharides found in the cell walls of Gram-negative bacteria, and are the most representative pyrogen. Bringing a transfusion, injectable drug, blood or the like that has been contaminated with an endotoxin into the human body has the potential to provoke severe side effects such as fever and shock. For this reason, there is a responsibility to manage the above-mentioned drugs and the like so as to prevent the contamination thereof with an endotoxin.

By the way, a hemocyte extract of a limulus (hereinafter, also referred to as "limulus amoebocyte lysate (LAL)") contains serine protease that is an enzyme activated by endotoxin. When LAL reacts with endotoxin, a coagulogen present in LAL is hydrolyzed into coagulins by an enzyme cascade by the serine protease activated according to the amount of endotoxin, and the coagulins are associated to form an insoluble gel. With the use of this property of LAL, it is possible to detect endotoxin with a high sensitivity.

Furthermore, β-D-glucan is a polysaccharide that constitutes a cell membrane characteristic of fungi. Measurement of β-D-glucan is effective, for example, for screening of fungus infections caused by a variety of fungi including not only fungi that are frequently observed in general clinical practices, such as Candida, Spergillus, and Cryptococcus, but also rare fungi.

Also in the measurement of β-D-glucan, by using the property of the limulus amoebocyte lysate to coagulate (coagulate to form a gel) by β-D-glucan, the β-D-glucan can be detected with a high sensitivity.

Turbidimetric assay is one method for detecting or measuring the concentration of a physiologically active substance of biological origins (hereinafter also called "a predetermined physiologically active substance") that can be detected using the hemocyte extract components of limuli, such as these endotoxins and β-D-glucans. This is a method for analyzing by temporally measuring the turbidity of a sample caused by the formation of gel from the reaction between LAL and the predetermined physiologically active substance when a sample in which the predetermined physiologically active substance needs to be detected or the concentration thereof needs to be measured (hereinafter simply called "measuring the predetermined physiologically active substance") is mixed with LAL and the resulting liquid mixture is allowed to stand.

When the turbidimetric assay described above is used to measure the predetermined physiologically active substance, the liquid mixture of the measurement sample and LAL is generated in a glass-made measuring cell that has been subjected to dry heat sterilization. The gelation of the liquid mixture is also optically measured from the exterior.

There has also been a proposal for a laser light scattering particle counting method (hereinafter also simply called the light scattering method) in which the presence of the predetermined physiologically active substance in a sample can be measured in a short period of time based on the intensity of laser light scattered by gel particles, or alternatively the intensity of light passing through the liquid mixture, when gel fine particles are generated by using, for example, a magnetic stirring bar to stir a liquid mixture of the measurement sample and LAL. A stirring turbidimetric assay has also been proposed, where a reaction is promoted by homogenizing a gelation state in a liquid mixture by stirring a measurement sample, which is only one aspect of the turbidimetric assay. Furthermore, there are others such as a colorimetric assay for using a synthetic substrate colorized by hydrolysis from an enzymatic cascade in order to optically measure the colorization.

Herein, it is a point of difference that light transmittance is detected in the turbidimetric assay and the stirring turbidimetric assay described above, while generated particles are detected in the light scattering method and changes in absorbance due to colorization are detected in the colorimetric assay. However, in each of these methods, the basis for the decision is made to be a threshold method for calculating the time from when the measurement sample and LAL are mixed together to when the intensity of transmitted light from the liquid mixture, or alternatively the intensity or the number of peaks in the scattered light, exceeds a threshold value.

In such cases, the variation in the timing in which the measurement sample and the LAL are mixed exerts an effect on the measurement precision of the concentration of the predetermined physiologically active substance. Conventionally, after a measurer has mixed the measurement sample and LAL in a container, the time measurement is initiated when the measuring vessel containing the liquid mixture is installed in the measurementdevice. Accordingly, failuretomanagethe relationship between the actual mixing timing of the measurement sample and LAL by the measurer and the measurement initiation time (or the analysis initiation time) often exerts an effect on the measurement precision of the concentration of the predetermined physiologically active substance.

### PRIOR ART DOCUMENTS

### PATENT DOCUMENTS

Patent Document 1: Japanese Patent No. 3322282
Patent Document 2: Japanese Patent Application Laid-Open No. 2004-239813
Patent Document 3: Japanese Patent No. 3283078

### DISCLOSURE OF THE INVENTION

### PROBLEM TO BE SOLVED BY THE INVENTION

The present invention has been made in light of the problems described above, and has the following points as the objective thereof. That is, the objective is to provide a technology capable of improving measurement precision by reducing the variation in the relationship between the mixture time of the sample and reagent and the time of initiating the measurement or analysis of the optical properties, in an optical reaction measurement device for measuring the presence or absence, or the extent of progression, of a reaction between a sample and reagent, based on temporal changes in the optical properties of the liquid mixture of a sample and reagent.

### MEANS FOR SOLVING THE PROBLEMS

The greatest feature in the present invention is that an optical reaction measurement device for measuring the presence or absence, or extent of progression, of a reaction between a sample and reagent based on temporal changes in the optical properties of the liquid mixture of a sample and reagent has a predetermined preparation period for generating the liquid mixture of the sample and reagent and preparing the same for measurement, such that when the measurement device is switched on, the time until the initiation time of the preparation period is continuously broadcast to the operator, and the fact that the preparation period has been initiated is also continuously broadcast to the operator, in order for analysis of the optical properties of the liquid mixture to be initiated from the end time of the preparation period.

More specifically, there is provided an optical reaction measurement device which measures the presence or absence, or extent of progression, of a reaction between a sample and reagent based on temporal changes in an intensity of scattered light or transmitted light, or in a value obtained by subjecting the intensity thereof to a given mathematical operation, as calculated by a calculation means, the device including:
a retaining means for retaining a sample cell containing a liquid mixture of a sample and a given reagent that reacts to the sample;
a light irradiation means for irradiating the liquid mixture in the sample cell with light;
a light intensity detection means for receiving scattered light or transmitted light of the light radiated onto the liquid mixture by the light irradiation means so as to detect the intensity of the scattered light or transmitted light; and
the calculation means for calculating temporal changes in the intensity of the scattered light or transmitted light detected by the light intensity detection means, or in a value obtained by subjecting the intensity thereof to a given mathematical operation, the device further including:
   a timer that, when an operator sets a switch to ON, initiates a count for a time elapsed since a point in time when the switch has been set to ON;
   a time broadcasting means for continuously broadcasting to the operator the time until an initiation time of a preparation period, in which a length of the preparation period is predetermined as the period for generating the liquid mixture by mixing the sample and reagent and then preparing the same for measurement, and the initiation time of the preparation period is taken as a time a given period of time has elapsed since the point in time when the switch has been set to ON;
   a preparation period initiation broadcasting means for broadcasting to the operator the initiation of the preparation period, wherein
   the presence or absence, or extent of progression, of the reaction between the sample and reagent is measured based on the temporal changes in the intensity of the scattered light or transmitted light, or in the value obtained by subjecting the intensity thereof to a given mathematical operation, which are calculated by the calculation means, after the end time of the preparation period.

That is, in the present invention, the preparation period for making the liquid mixture generated by mixing the sample and reagent ready for measurement is set to be initiated after a given period from the time when the switch is set to ON. The length of the preparation period is also set in advance. Further, after the switch is set to on, the time until the initiation time of the preparation period is continuously broadcast to the operator by a time broadcasting means. The operator is thereby able to prepare both physically and mentally for the advent of the preparation period.

Moreover, in the present invention, since the fact that the preparation period has begun is also broadcast to the operator by a preparation period initiation broadcasting means, the operator generates the liquid mixture by mixing the sample and reagent along with the initiation time of the preparation period, and is thereby able to quickly prepare the same for the measurement of optical properties.

In addition, in the present invention, the presence or absence, or the extent of progression, of a reaction is measured based on temporal changes in the intensity of the scattered light or transmitted light calculated by a calculation means, or in the value obtained by subjecting the intensity to given mathematical operations, after the end time of the preparation period. Accordingly, the end time of the preparation period can be taken as the initiation time for analyzing the optical properties of the liquid mixture. The start time and completion time of the operation (hereinafter simply called the "mixing operation") in which the operator prepares for the measurement of the liquid mixture generated by mixing the sample and reagent, and the start time for the analysis of the optical properties can thereby be supplied from the initiation time to the end time of the preparation period. As a result, it is possible to reduce the variation in these relationships.

The actual mixing time of the sample and reagent can thereby be accurately matched with the initiation time for the analysis of the optical properties, making it possible to enhance the accuracy for measuring the absence or presence, or extent of progression, of the reaction between the sample and reagent.

In the present invention, the time broadcasting means may also broadcast the time until the initiation of the preparation period to the operator by means of a countdown display.

By so doing, the operator can more clearly recognize the remaining time until the advent of the initiation time of the preparation period by means of the countdown display, and match the initiation time for the mixing operation with the initiation time of the preparation period with greater accuracy. It is also possible to more reliably reduce the variation between the actual mixing time of the sample and reagent and the initiation time for analyzing the optical properties of the liquid mixture.

In the present invention, it may also be possible to adjust the given time until the initiation time of the preparation period from the time when the switch is set to ON, and the length of the preparation period.

By so doing, it is possible to appropriately set the initiation time and length of the preparation period depending on the type of the sample and reagent, on the content of the mixing operation, and on the operator. It is thereby possible to more reliably reduce the variation in the relationship between the actual mixing time of the sample and reagent, and the initiation time for analyzing the optical properties thereof. It is also further possible to appropriately adjust the time difference between the actual mixing time of the sample and reagent, and the initiation time for analyzing the optical properties thereof.

Also, in the present invention, the retaining means is able to retain a plurality of sample cells,
the retaining means is able to independently measure the absence or presence, or extent of progression, of the reactions between the sample and reagent for the liquid mixture in the plurality of sample cells,
the length of the given time from the point when the switch is set to ON to the initiation time of the preparation period, and of the preparation period, is established at the same value for the liquid mixture in the plurality of sample cells, and
when the switch is set to ON, the time broadcasting means and the preparation period initiation broadcasting means may be made to actuate for the liquid mixture in the plurality of sample cells.

Accordingly, the operator only has to finish creating the liquid mixture by mixing the sample and reagent, introducing the liquidmixture into a plurality of sample cells, and installing onto the retaining means (has to terminate the mixing operation), during the preparation period. Therefore, the operator can focus on a simpler operation and perform a measurement of the presence or absence, or extent of progression, of the reaction between the sample and reagent for a plurality of liquid mixtures, more easily.

Also, in the present invention, the sample is a sample targeted for the detection of endotoxin or β-D-glucan, or for the measurement of the concentrations thereof, and
the given reagent may be LAL, which is the hemocyte extract of limuli.

Accordingly, it is possible to more accurately detect, or measure the concentrations of, endotoxin, which is the most typical pyrogen, and it is thus possible to reduce the risk of side effects provoked by endotoxin contamination in the body. It is similarly possible to more accurately detect, or measure the concentration of, β-D-glucan, and it is thus possible to more accurately screen for a broad range of fungus infections including not only fungi commonly seen in general clinical pathology such as Candida, Spergillus, and Cryptococcus, but also rare fungi.

In such cases, the time measurement value, which is associated with the intensity of scattered light or transmitted light detected by the light intensity detection means or with a value in which the intensity thereof is subjected to a given mathematical operation, may be taken as the elapsed time from the initiation time of the preparation period.

That is, the time information associated with the measurement value of the intensity of scattered light or transmitted light or with the value in which the measurement value is subjected to a given mathematical operation is taken as the elapsed time from the initiation time of the preparation period. By so doing, it is possible to analyze the temporal changes in the intensity of the scattered light or transmitted light or in the value created by subjecting the intensity to a given mathematical operation, with the assumption that the sample and reagent having been mixed together at the initiation time of the preparation period (which is the zero-count time, when the time from the switch ON until the initiation time of the preparation period is displayed as a countdown).

Herein, in the present invention, given that the operator prepares both mentally and physically for the mixing operation from before initiation time of the preparation period, the closest time to the actual mixing time of the sample and reagent is thought to be not the termination but rather the initiation time of the preparation period. Therefore, the time measurement value associated with the intensity of the scattered light or transmitted light detected by the light intensity detecting means or with a value in which the intensity thereof is subjected to a given mathematical operation is taken as the elapsed time from the initiation time of the preparation period. This makes it possible to bring the time information, which is for example, the start time of the reaction between endotoxin and LAL, to a value that is closer to the actual mixing time.

Also, the present invention may be an optical reaction measurement method, including:
irradiating a liquid mixture of a sample and a given reagent that reacts to the sample with light;
detecting an intensity of scattered light or transmitted light of the light radiated onto the liquid mixture; and
measuring the presence or absence, or extent of progression, of a reaction between the sample and reagent based on temporal changes in the detected intensity of the scattered light or transmitted light, or in a value obtained by subjecting the intensity to a given mathematical operation, the method further including:
   an intention indicating step for indicating an intention to initiate measurement after a measurer mixes the sample and reagent to prepare the liquid mixture;
   a time broadcasting step for continuously broadcasting to the measurer the time until the initiation of a preparation period, in which a length of the preparation period is predetermined as the period for generating the liquid mixture by mixing the sample and the reagent and then preparing the same for measurement, and the initiation time of the preparation period is taken as the time a given period of time has elapsed since the point in time when the intention indication has been performed; and
   apreparationperiodinitiationbroadcastingstep for broadcasting to the measurer the initiation of the preparation period, wherein
   the presence or absence, or extent of progression, of the reaction between the sample and reagent is measured based on changes in the intensity of the scattered light or transmitted light or in a value obtained by subjecting the intensity thereof to a given mathematical operation, after the end time of the preparation period.

Accordingly, after the measurer performs the mixing operation and indicates the intention to begin measuring the optical properties of the liquid mixture, it is possible to more clearly recognize the remaining time until the initiation time of the preparation period, and also to prepare for the mixing operation, both mentally and physically, until the initiation time of the preparation period. As a result, it becomes possible to perform the mixing operation more smoothly or more rapidly in accordance with the advent of the initiation time of the preparation period.

It is thereby possible to fit the start time and end time for the mixing operation by the measurer and the start time for analyzing the optical properties in between the initiation time and end time of the preparation period, and thus to reduce the variation of these relationships.

Further, the present invention may be configured such that
the presence or absence, or extent of progression, of the reaction between the sample and reagent is independently measured for a plurality of liquid mixtures,
the intention indication in the intention indicating step is the indication of the intention to prepare a plurality of liquid mixtures by mixing the sample and the reagent as well as to begin measuring the plurality of liquid mixtures,
the length of the preparation period and of the given time from the point in time when the intention is indicated until the initiation time of the preparation period is set to the same value for the plurality of liquid mixtures, and
the time broadcasting step and the preparation period initiation broadcasting step are executed for the plurality of liquid mixtures in the sample cells when the intention is indicated.

Accordingly, the measurer only has to finish introducing the liquid mixtures prepared by mixing the sample and reagent into a plurality of sample cells and then installing the same onto the retaining means, during the preparation period. The measurer is therefore able to focus on the simple operation, and it becomes possible to perform a measurement of the presence or absence, or extent of progression, of the reaction between the sample and reagent for the plurality of liquid mixtures, more easily.

Further, the present invention may be configured such that
the sample is a sample targeted for the detection of endotoxin or β-D-glucan, or for the measurement of the concentrations thereof, and
the given reagent may be LAL, which is the hemocyte extract of limuli.

Accordingly, it is possible to more accurately detect, or measure the concentrations of, endotoxin, which is the most typical pyrogen, and it is thus possible to reduce the risk of side effects provoked by endotoxin contamination in the body. It is similarly possible to more accurately detect, or measure the concentration of, β-D-glucan, and it is thus possible to more accurately screen for a broad range of fungus infections including not only fungi commonly seen in general clinical pathology such as Candida, Spergillus, and Cryptococcus, but also rare fungi.

Further, in such cases, the time measurement value associated with the intensity of scattered light or transmitted light, or with a value in which the intensity thereof is subjected to a given mathematical operation, may be taken as the time elapsed since the initiation time of the preparation period.

That is, the time information associated with the measurement value of the intensity of the scattered light or transmitted light, or with a value in which the measurement value is subjected to a given mathematical operation, is taken as the elapsed time from the initiation time of the preparation period.

Herein, in the present invention, given that the measurer prepares both mentally and physically for the mixture operation prior to initiation time of the preparation period, the closest time to the actual mixing time of the sample and reagent is thought to be not the end time but rather the initiation time of the preparation period. Therefore, the fact that the time information associated with the measurement value of the intensity of scattered light or transmitted light is taken as the elapsed time from the initiation time of the preparation period makes it possible to treat the time information, which is for example the start time of the reaction between endotoxin and LAL, as a value that is closer to the actual mixing time.

Note that in the above-described solutions to the problems of the present invention, every possible combination thereof can be used.

### ADVANTAGEOUS EFFECTS OF INVENTION

In the optical reaction measurement device of the present invention for measuring the presence or absence, or extent of progression, of the reaction between a sample and reagent based on the temporal changes in the optical properties of a liquid mixture of the sample and reagent, it becomes possible to reduce the variation in the relationships between the mixing time of the sample and reagent and the start time for analyzing the optical properties, thus improving measurement precision.

### BRIEF DESCRIPTION OF THE DRAWINGS

FIG. 1 is a diagram illustrating the schematic configuration of a light scattering particle measurement device in Example 1 of the present invention.
FIG. 2 is a diagram illustrating the internal configuration of the light scattering particle measurement device in Example 1 of the present invention.
FIG. 3 is a flowchart illustrating a preparation period control routine in Example 1 of the present invention.
FIG. 4 is a diagram illustrating a measurement screen displayed on a display unit in Example 1 of the present invention.
FIG. 5 is a diagram illustrating a condition setting screen displayed on the display unit in Example 1 of the present invention.
FIG. 6 is a diagram illustrating a measurement screen in Example 2 of the present invention.
FIG. 7 is a diagram illustrating the internal configuration of a turbidimetric measurement device in Example 3 of the present invention.

### BEST MODE FOR CARRYING OUT THE INVENTION

Amore detailed description is provided below, with reference to the drawings, for the optical reaction measurement device and optical reaction measurement method in the present invention. The measurement to which the present invention is to be applied, as described above, is a technique for measuring the presence or absence, or extent of progression, of the reaction between a sample and reagent by changing the optical properties of a sample by means of the reaction between the sample and reagent in order to then acquire the change in the optical properties.

An example of the above-mentioned technique may include, for example, the technique for measuring the concentration of water pollution components by using a dedicated colorizing reagent prepared in advance in order to measure the absorbance while the sample is colorized by being mixed with the reagent in a measurement container. Another example can be the immunoassay for optically detecting aggregates formed by a specific antigen-antibody reaction which is provoked by adding either an antibody for an antigen or an antigen for an antibody as a reagent for the reaction with the antigen or antibody in a specimen.

From among the various measurements to which the present invention can be applied, a description is provided in Examples below for the example of the technique for optically measuring the gelation of a liquid mixture caused by the reaction occurring when an LAL reagent is added to a sample containing a physiologically active substance of biological origins such as endotoxin or β-D-glucan.

The mechanism in which LAL reacts with endotoxin to generate a gel (hereinafter also referred to as the Limulus reaction) is well documented. That is, when endotoxin binds to C-factor, which is a serine protease in LAL, the C-factor is activated to become activated C-factor. The activated C-factor hydrolyses B-factor, which is another serine protease in LAL, to activate the same and make activated B-factor. The activated B-factor immediately hydrolyzes the precursors of coagulase in LAL to make coagulase, and furthermore, the coagulase hydrolyzes the coagulogen in LAL to generate coagulins. Further, it is believed that the generated coagulins are associated with each other to further generate an insoluble gel, which gelates the entire LAL involved therein.

In addition, similarly, when β-D-glucan binds to G-factor in LAL, the G-factor is activated and becomes activated G-factor. The activated G-factor makes coagulase by hydrolyzing the precursors of coagulase in the LAL. As a result, in the same manner as the reaction between endotoxin and LAL, coagulins are generated, and the association of the generated coagulins with each other further generates an insoluble gel.

Such an enzyme cascade reaction has an extremely strong amplification effect for activation by linking a subsequent cascade, even with only a small amount of activation factor. Thus, according to the method for measuring a predetermined physiologically active substance using LAL, it becomes possible to detect the predetermined physiologically active substance, on the order of very small sub-picogram amounts per mL.

Examples of a measurement method with which it is possible to quantify a predetermined physiologically active substance such as endotoxin or β-D-glucan include the turbidimetric assay, stirringturbidimetricassay, and light scattering method as described above. In these measurement methods, aggregates of coagulins generated by the LAL enzyme cascade reaction are taken in the former two methods as the turbidity of the sample, and in the latter method as the micro particles of the gel generated within the system, for detection using an optical technique that enables measurement to a high degree of sensitivity.

Based on the facts that no special reagents are required and that there is a broad range of measurable concentrations for predetermined physiologically active substances, the turbidimetric assay has been evaluated as having a better user-friendliness in the field. However, because gelation only occurs once the concentration of coagulin reaches a concentration above a certain degree, in the turbidimetric assay it is necessary to wait until gelation occurs in order to detect the predetermined physiologically active substance. For this reason, the measurement period is short when there is a high concentration of the predetermined physiologically active substance, because the gelation begins immediately after the coagulin is generated with a required sufficient concentration. On the other hand, when there is a low concentration of the predetermined physiologically active substance, time is required for the coagulin to reach the required concentration for gelation, and thus the measurement period ends up being long. In this respect, in the stirring turbidimetric assay, stirring the liquid mixture of the predetermined physiologically active substance and the LAL is intended to shorten the measurement period by promoting the reaction between the two.

The light scattering method is an improvement over the turbidimetric assay in that particles are detected by a laser rather than gelation when the sample is stirred, making it possible to greatly reduce the measurement time when compared to the turbidimetric assay. The turbidimetric assay and stirring turbidimetric assay, as well as the light scattering method, share the fact that the time at which a certain threshold is exceeded is treated as the start time for the reaction, in spite of the fact that the physical amount to be observed differs.

### [Example 1]

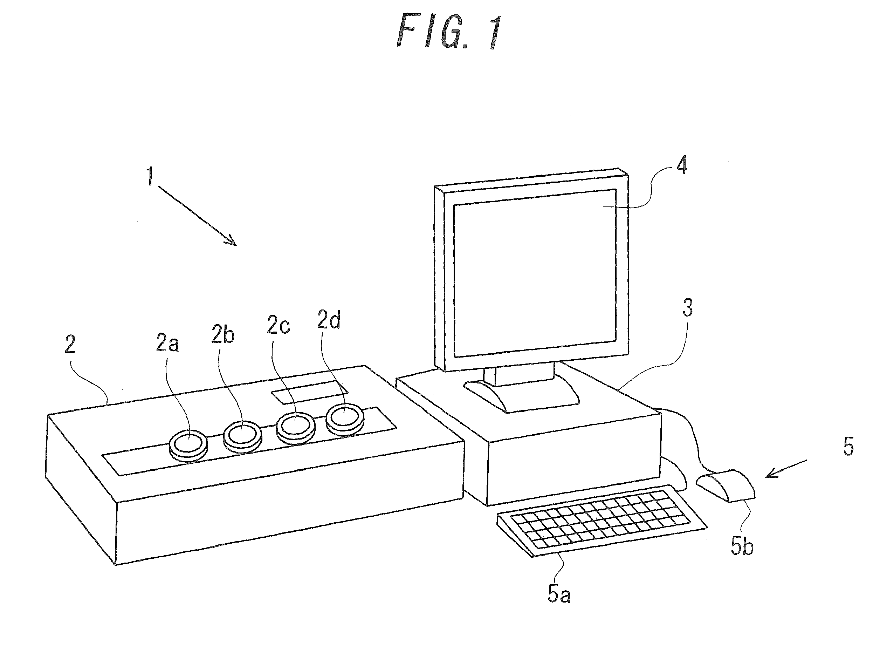

Next, a description will be provided for a measurement device using the light scattering method, as Example 1. FIG. 1 illustrates the outer appearance of a light scattering particle measurement device 1 serving as the device for measuring endotoxin in this Example.

The light scattering particle measurement device 1 is provided with a main body 2 and a calculation unit 3, a display unit 4, and an input unit 5 including a keyboard 5a and a mouse 5b for various types of input into the calculation unit 3. The main body 2 is configured such that four cell holders 2a to 2d are provided so as to hold four sample cells, enabling the measurement of four channels for the samples housed within the sample cells. In this Example, the cell holders 2a to 2d correspond to the retaining means.

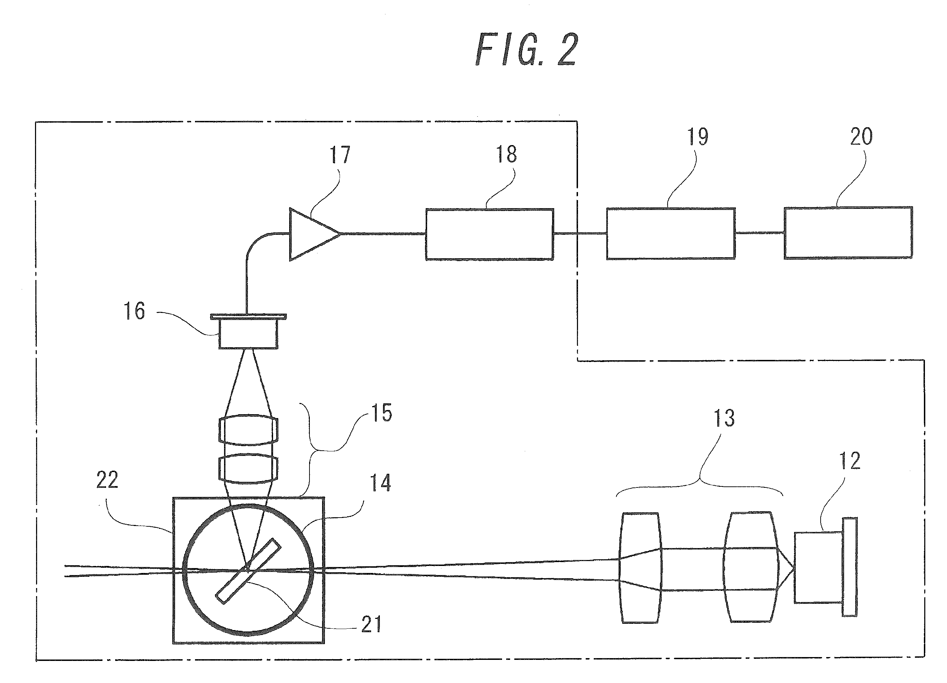

Next, the internal configuration of the light scattering particle measurement device 1 will be described with reference to FIG. 2. Note that for the sake of simplicity, in FIG. 2, the light scattering particle measurement device is described as corresponding to one channel. A laser light source is used as a light source 12 to be used in the light scattering particle measurement device 1, but other light sources may be used, such as an ultra-high brightness LED. The light radiated from the light source 12 is narrowed by an incident optical system 13 so as to be incident on a sample cell 14. The sample cell 14 retains the liquid mixture of the sample in which the endotoxin needs to be measured and the LAL reagent. Light incident on the sample cell 14 is scattered by the particles in the liquid mixture (the measurement targets, such as coagulogen monomer and coagulogen oligomer).

A projection optical system 15 is arranged on the side of the incident optical axis of the sample cell 14. A light receiving element 16, which receives the scattered light that has been scattered by the particles in the liquid mixture inside the sample cell 14 and narrowed by the projection optical system 15 in order to convert the same to electrical signals, is also arranged on an extension of the optical axis of the projection optical system 15. The light receiving element 16 is electrically connected to an amplification circuit 17 that amplifies the electrical signals photoelectrically converted by the light receiving element 16, and to a filter 18 for removing noise from the electrical signals amplified by the amplification circuit 17. The light receiving element 16 is further electrically connected to a calculation device 19 that calculates the number of gel particles from the peak number of electrical signals after the noise has been removed in order to further decide the reaction start time and thus derive the concentration of endotoxin, and to a display device 20 that displays the results. The main body 2 contains the portion enclosed in the dashed line in the drawing, the calculation unit 3 contains the calculation device 19, and the display unit 4 contains the display device 20.

Also, the sample cell 14 is provided with a stirring bar 21 for stirring the liquid mixture serving as the sample, by rotation when an electromagnetic force is exerted from the exterior. The exterior of the sample cell 14 is also provided with a stirrer 12. It is thereby possible to adjust the presence or absence of stirring and the speed of the stirring.

Herein, the light source 12 and the incident optical system 13 correspond to the light illumination means. The projection optical system 15 and the light receiving element 16 correspond to the light intensity detectionmeans. The calculation device 19 corresponds to the calculation means.

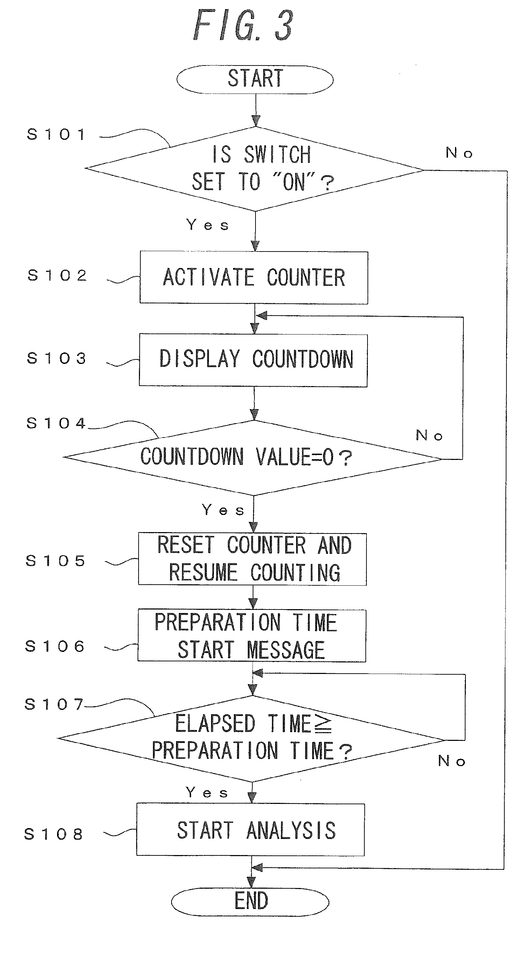

Next, a description will be provided for the control of the preparation time upon beginning of measurement with the light scattering particle measurement device 1 described above (the preparation time in this Example corresponds to the preparation period). FIG. 3 shows the flowchart of the preparation time control routine in this Example. This routine is a flow executed by a CPU, which is not shown, within the calculation unit 3.

When this routine is executed, first, in step S101, a determination is made as to whether the switch described below has been set to ON in order to indicate the intention to perform light scattering particle measurement by the operator or measurer (hereinafter simply referred to as the operator). Herein, the routine is temporarily terminated as is when a determination is made that the switch has not been set to ON. On the other hand, when the determination is made that the switch has been set to ON, the processing proceeds to S102.

In S102, the counter is activated to measure the time elapsed since when the switch is set to ON. The processing proceeds to S103 after the end of S102. In S103, the countdown is displayed. As will be described later, in this Example, the initial value of the countdown is set to ten seconds. Therefore, as the time decreases from ten seconds, the remaining time until the initiation time for the preparation time is displayed. The countdown display may be displayed on a screen of the display unit 4, or may alternatively be displayed on a special indicator that is provided on the main body 2. The processing proceeds to S104 after the end of S103.

In S104, a decision is made whether or not the countdown value has reached zero. Herein, a decision that the countdown value has not yet reached zero prompts a return to before S103, and the countdown value continues to be displayed. On the other hand, when a decision is made that the countdown value has reached zero, the processing proceeds to S105.

In S105, the counter is reset and resumes counting the time elapsed since when the preparation time has begun. The processing proceeds to S106 after the end of S105.

In S106, a message is displayed to the effect that the preparation time has begun. The message, like the countdown display, may be displayed on the screen of the display unit 4 or may be displayed on a special indicator that is provided on the main body 2. The message may also be broadcast using audio. Furthermore, it would also be possible to display (by substitution) the message when the countdown display reaches zero. The processing proceeds to S107 after the end of S106.

In S107, a decision is made whether the time elapsed since the start time of the preparation time is greater than the predetermined length of the preparation time. In this Example, as will be described below, the length of the preparation time is set to 30 seconds, and therefore an elapsed time less than 30 seconds prompts a return to before S107 and the repeated execution of S107. On the other hand, when the elapsed time is 30 seconds or greater, the processing proceeds to S108.

In S108, the analysis of measurement data is initiated using the light scattering particle measurement method. In particular, the peak value of the scattered light intensity detected by the light receiving element 16 is counted in order to begin calculating the total number of gel particles in the liquid mixture. This routine is completed once the processing for S108 ends. Note that another routine is performed to control the light scattering particle measurement itself, but the description thereof is omitted herein. Note also that in the above-described preparation time control routine, the CPU of the calculation unit 3 executing the processing of S102 to S104 constitutes the time broadcasting means in this Example. Further, the CPU of the calculation unit 3 executing the processing of S105 to S106 constitutes the preparation period initiation broadcasting means in this Example. In the above-described preparation time control routine, the processing of S103 and S104 corresponds to the time broadcasting step. Further, the processing of S106 corresponds to the preparation period initiation broadcasting step.

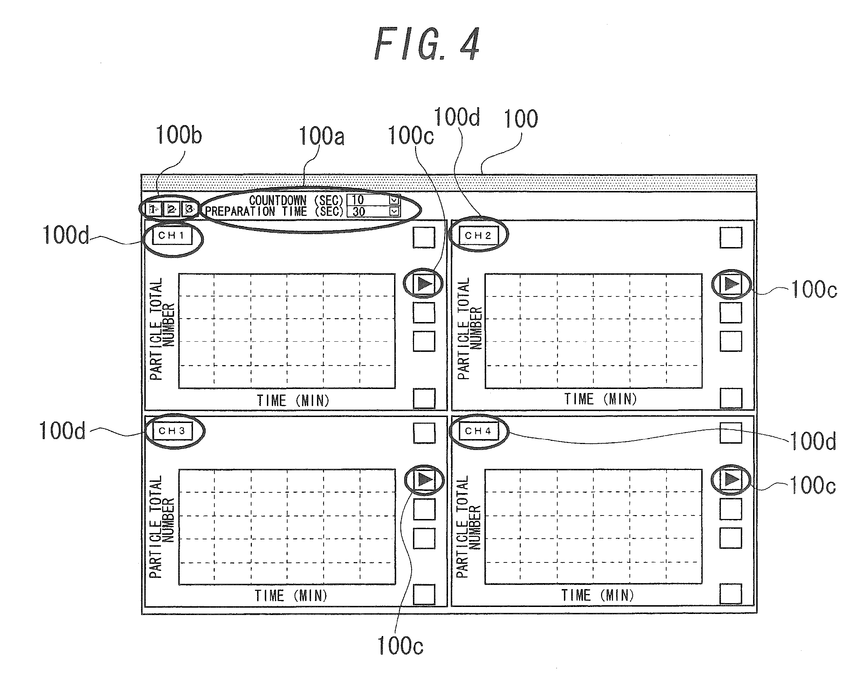

FIG. 4 illustrates a measurement screen 100 displayed at the time of measurement, in the display unit 4 of the light scattering particle measurement device 1 of this Example. This screen displays respective graphs with the total number of particles, which is calculated by the calculation device 19 based on the intensity of scattered light from the liquid mixture inside the sample cells 14 being retained by the cell holders 2a to 2d, in the measurement system for channel 1 to channel 4.

Before beginning measurement, a countdown timer (display: the countdown) and an analysis start timer (display: preparation time) are set in a timer setting unit 100a of this screen. In FIG. 4, the set value of the countdown timer is ten seconds, and the preparation time until the analysis start time is 30 seconds. Note that in this screen, the operator is also able to independently select a channel to be used from among the four channels and to independently start the countdowns, or simultaneously start countdowns for a plurality of preset channels. Herein, when independently selecting a channel to be used and independently starting a countdown, the operator clicks on the start button 100c for the channel to be used after having prepared the sample and LAL reagent for measuring the concentration of endotoxin. Further, when starting countdowns for a plurality of channels together, the operator clicks on the button for a simultaneous start button unit 100b after having prepared the sample and LAL reagent for measuring the concentration of endotoxin.

Then, countdown values are displayed in the bottom of a channel display unit 100d for the channels to be used of the measurement screen 100. These countdown values will decrease from ten seconds by a count of one. The operator is encouraged by the countdown display to begin the preparation operation at the timing when the countdown value reaches zero. Taking the time when the countdown value reaches zero as a cue, the operator uses the time of 30 seconds that have been set as the preparation time in order to mix the previously prepared sample and LAL reagent and then stir the same using a centrifuge or the like so as to prepare the liquid mixture. The liquid mixture is then poured into a required number of sample cells 14, and the required number of sample cells 14 into which the liquid mixture has been introduced are then installed into the corresponding cell holder of the cell holders 2a to 2d. That is, the operator has 30 seconds set as the preparation time for completing "mixing the sample and LAL reagent", "pouring (dispensing) into the sample cells 14", and "installing the sample cells 14 into the cell holders 2a to 2d".

Thereafter, at the point in time when 30 seconds of the preparation time have elapsed, the total number of gel particles inside the liquid mixture is calculated based on the intensity of scattered light detected by the light receiving element 16, by means of an application that has been loaded onto the calculation unit 3. Moreover, variations in the total number of gel particles are displayed on the measurement screen 100 for each channel, and also stored in a memory (not shown). Note that the ten seconds acting as set value for the countdown timer as described above correspond to the given period of time in this Example. The step in which the operator clicks on the button of the simultaneous start button unit 100b corresponds to the intention indicating step in this Example. The total number of gel particles in the liquid mixture as calculated based on the intensity of scattered light detected by the light receiving element 16 corresponds to the value in which the intensity of scattered light has been subjected to a given mathematical operation in this Example.

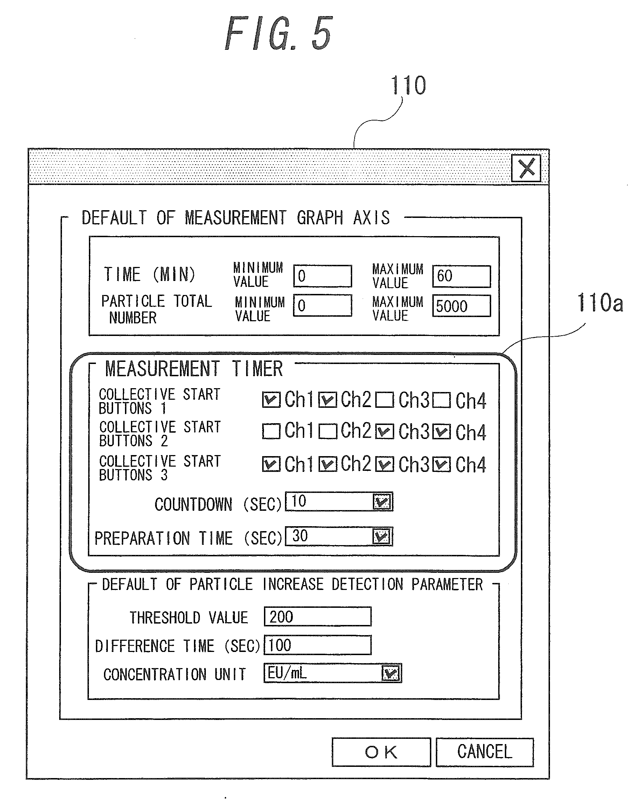

FIG. 5 illustrates a condition setting screen 110 in this Example for when the measurements by a plurality of channels are started at the same time. A measurement timer setting unit 110a of the condition setting screen 110 enables setting combinations of channels allocated to one to three buttons in the simultaneous start button unit 100b in the measurement screen 100. In this example, clicking on the button 1 of the simultaneous start button unit 100b in the measurement screen 100 begins the countdown of channels 1 and 2, the count for the preparation time and the measurement of the number of particles. Similarly, clicking on the button 2 initiates the countdown for channels 3 and 4, the count for the preparation time and the measurement of the number of particles, while clicking on the button 3 initiates the countdown for all the channels 1 to 4, the count for the preparation time and the measurement of the number of particles.

As has been described above, in this Example, the countdowns are initiated after the start button 100c for the channels to be used or the button of the simultaneous start button unit 100b is clicked on by the operator, and the operator has the time during the countdowns to prepare both mentally and physically for the operation of mixing the sample and LAL reagent including preparation of the sample and LAL reagent.

Then, after the countdown value reaches zero and the preparation time is begun, the operator has the 30 seconds during the preparation time to complete mixing the sample and LAL reagent, transferring to each sample cell 14, and installing onto each cell holder.

Thereby, first, the fact that the countdown is performed prior to initiation time of the preparation time allows the operator to focus on preparing for the measurement preparation operations such as mixing the sample and LAL reagent, transferring to each sample cell 14, and installing onto each cell holder. In addition, setting the preparation time makes it possible to prompt the operator to finish the measurement preparation operations, including mixing the sample and LAL reagent, transferring to each sample cell 14, and installing onto each cell holder, within the given time period.

Note that in this Example, the time data associated with the particle total number data is taken as the start time for the preparation time (the point in time at which the countdown value reaches zero). Therefore, the time standard for the measurement data is clearer, and the time standard can be brought as close as possible to the actual time at which the sample and LAL reagent have beenmixed. Moreover, the analysis itself of the actual particle total number data is begun at the time when the preparation time ends, which makes it possible to carry out the analysis using only the data from the state in which the liquid mixture of the sample and LAL reagent has been introduced into the sample cells and then entirely installed onto the cell holders. The result is that the variation in the detection of endotoxin or measurement of the concentration thereof in each channel can be suppressed, thus improving the measurement precision. Note that it would be possible to perform the calculation and storage itself of the particle total number data from the start time of the preparation time.

Furthermore, in Example described above, because it is possible to preset combinations of channels to be measured so as to correspond to each of the buttons 1 through 3 of the simultaneous start button unit 100b, there is no need to set channels at each measurement when measuring with a plurality of channels at the same time, enabling the use of a single click to initiate measurement for three different combinations.

Note that in the above-described Example 1, once the operator has clicked on the simultaneous start button unit 100b, a countdown is displayed for the ten seconds up until the preparation time is begun, but this display need not necessarily be a countdown display. For example, when it is known that the preparation time will begin ten seconds after the simultaneous start button unit 100b is clicked, then it is obvious that a count-up may also be displayed from second 1 until second 10. Further, in the measurement screen 100, the message that is broadcast to the operator combines both the fact that the displayed countdown value has reached zero and that the preparation time has begun, but it would also be possible to carry out processing such as opening a window for displaying an additional preparation time start message.

### [Example 2]

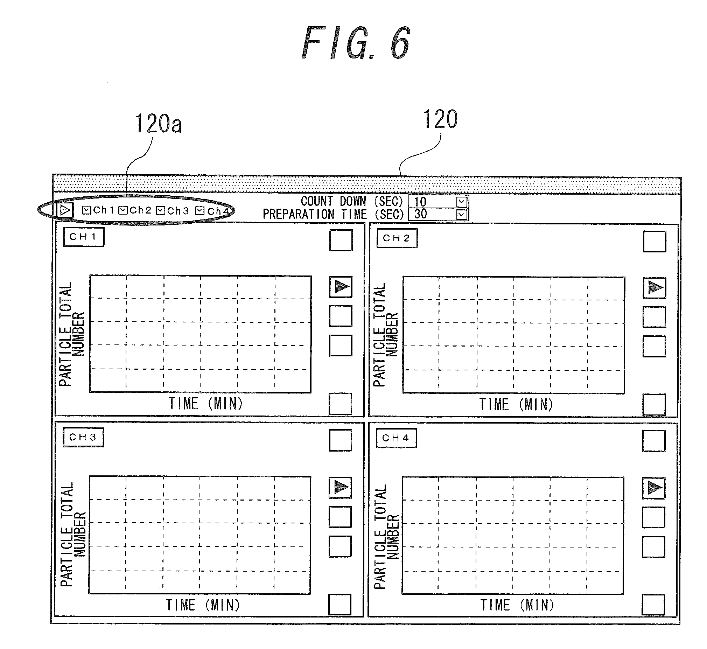

Next, a description will be provided for Example 2 of the present invention. FIG. 6 illustrates a measurement screen 120 according to this Example. In this Example as well, the fundamental operation is identical to what is described in Example 1. However, by contrast to the fact that in Example 1 combinations of operation channels are pre-assigned to the buttons 1 to 3 of the simultaneous start button unit 100b in the condition setting screen 110, in Example 2 the combinations of channels for simultaneous start are made to be set for each measurement in a start button unit 120a.

Accordingly, since the combinations of channels for which measurement is to begin at the same time are set for each measurement whenever measurement by a plurality of channels is to be performed together, there is no need to understand the relationship between the button numbers of the simultaneous start button unit 100b and the combinations of operation channels, enabling ad hoc adaptations to measurements for a broad range of applications.

### [Example 3]

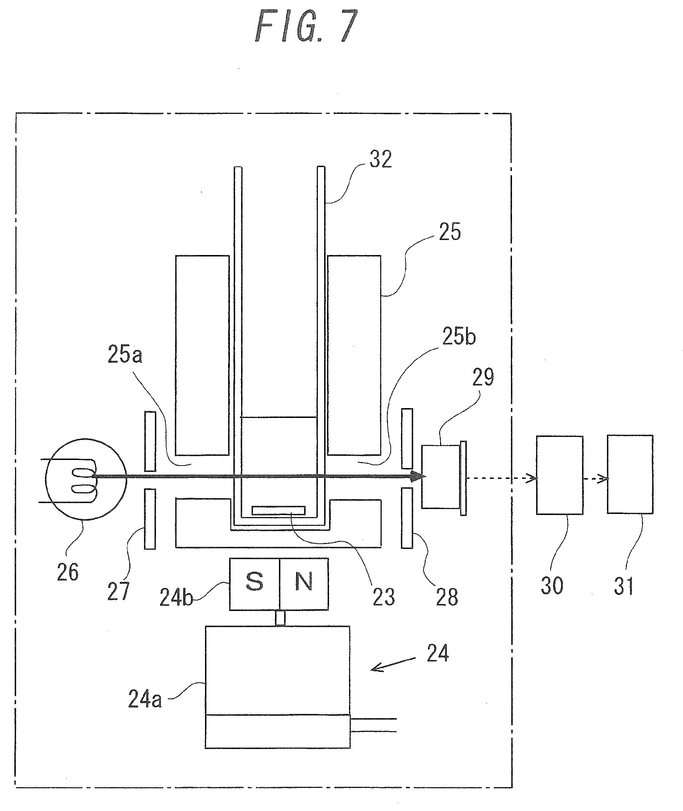

Next, a description will be provided for a turbidimetric measurement device serving as the device for measuring endotoxin to which the present invention can be applied, as Example 3. The outer appearance of the turbidimetric measurement device in this Example is equivalent to what is illustrated in FIG. 1, and it is still possible to measure four channels. FIG. 7 illustrates the internal configuration of the turbidimetric measurement device. The portion shown with the dashed line in the drawing is provided for a total of four respective channels, but herein, for the sake of simplicity, a description is provided for only one channel. The turbidimetric measurement device of this Example measures endotoxin using the stirring turbidimetric assay. In this Example, the liquid mixture of a sample containing endotoxin and the LAL reagent is introduced into a sample cell 32. A warmer 25 is provided so as to surround the sample cell 32. The interior of this warmer 25 is provided with a heating wire not shown in the drawing, where a current is passed through the heating wire in order to keep warm the sample cell 32 at about 37°C. Note that the warmer 25 in the present Example acts as one part of the retaining means. A stirring bar 23 made of stainless steel is provided within the sample cell 32. The stirring bar 23 rotates inside the sample cell 32 by the action of a stirrer 24 installed in the bottom of the sample cell 32. That is, the stirrer 24 includes a motor 24a and a permanent magnet 24b provided on the output shaft of the motor 24a. Also, the permanent magnet 24b is rotated when an electrical current is passed through the motor 24a. Because the magnetic field from this permanent magnet 24b is rotated, the stirring bar 23 is rotated by the action of the rotating magnetic field.

Note that in the turbidimetric measurement device of this Example, a light source 26 serving as the light illumination means and a light receiving element 29 serving as the light intensity detection means are provided. The light emitted from the light source 26, after having passed through an aperture 27, passes through a light incident hole 25a provided on the warmer 25 and is incident on the sample inside the sample cell 32. Light that has passed through the sample inside the sample cell 32 is emitted through an emission hole 25b provided on the warmer 25 and passes through an aperture 28 so as to irradiate the light receiving element 29. A photoelectric signal corresponding to the intensity of the received light is output by the light receiving element 29. The output of this photoelectric signal is input into a calculation device 30 serving as the calculation means provided in the calculation unit 3. In the calculation device 30, temporal changes in the intensity of the transmitted light are calculated according to a pre-loaded program (algorithm). Also, the reaction start time is determined and the concentration of endotoxin is derived. Note that the intensity of transmitted light as calculated by the calculation device 30 is input into and appropriately displayed on a display device 31 provided on the display unit 4.

The present invention described in Examples 1 and 2 can be applied substantially as is for such a turbidimetric measurement device as well. In such a case, the vertical axis of the graph in the measurement screens 100 and 120 becomes the intensity of transmitted light. Further, portions other than the measurement timer setting unit 110a in the condition setting screen can be appropriately modified.

Note that in the Example described above, the present invention has been applied to measuring the presence or absence, or extent of progression, of a reaction between a sample including endotoxin and an LAL reagent, but the present invention can of course be applied to the reaction between a sample including β-D-glucan and an LAL reagent. Also, the present invention may be applied to measuring the presence or absence, or extent of progression, of a reaction between other types of samples and reagents. For example, it is also possible to apply the present invention to a technique for measuring the concentration of water contamination components by using a dedicated colorizing reagent prepared in advance in order to colorize a sample by mixing with a reagent in a measuring container and then measure the absorbance. The present invention can of course also be applied to an immunoassay for optically detecting aggregates formed by an antigen-antibody reaction when a specific antigen-antibody reaction is incited by adding an antibody for an antigen or an antigen for an antibody as a reagent to react with the antigen or antibody in a specimen.

### [Reference Example]

Next, as a reference example of the present invention, mention will be made of an example in which a foot switch is set to ON at the initiation time of the preparation time by the operator, as a point of difference from Examples described above in which the start button is clicked on in order to carry out a countdown for the time until the initiation time of the preparation time. In this reference example, there is no countdown function as described in Examples above, but rather a foot switch is set to ON at the point in time when the operator has prepared both physically and mentally for the mixing operation of the sample and LAL reagent. Then, when the foot switch is set to ON, a timer for a predetermined channel is started and the count for the preparation time is commenced. The fact that the analysis of the measured values is begun after the preparation time has elapsed is identical to Examples described above.

According to the reference example, when the sample and LAL reagent are mixed, the foot switch can be set to ON when a judgment is independently made as to the timing that allows for sufficient mental and physical preparation. There is also no need to redo everything from the start when it is desirable to retry due to troubles during the countdown or preparation time.

### DESCRIPTION OF REFERENCE NUMERALS

- 1: light scattering particle measurement device
- 2: main body
- 2a-2d: cell holders
- 3: calculation unit
- 4: display unit
- 5: input unit
- 5a: keyboard
- 5b: mouse
- 12: light source
- 13: incident optical system
- 14: sample cell
- 15: projection optical system
- 16: light receiving element
- 17: amplification circuit
- 18: noise removal filter
- 19: calculation device
- 20: display device
- 21: stirring bar
- 22: stirrer
- 23: stirring bar
- 24: stirrer
- 24a: motor
- 24b: permanent magnet
- 25: warmer
- 25a: light incident hole
- 25b: emission hole
- 26: light source
- 27: perture
- 28: aperture
- 29: light receiving element
- 30: calculation device
- 31: display device
- 32: sample cell
- 100: measurement screen
- 100a: timer setting unit
- 100b: simultaneous start button unit
- 100c: start button
- 100d: channel display unit
- 110: condition setting screen
- 110a: measurement timer setting unit
- 120: measurement screen
- 120a: start button unit

## Claims

1. An optical reaction measurement device which measures the presence or absence, or extent of progression, of a reaction between a sample and reagent based on temporal changes in an intensity of scattered light or transmitted light, or in a value obtained by subjecting the intensity thereof to a given mathematical operation, as calculated by a calculation means, the device comprising:
a retaining means for retaining a sample cell containing a liquid mixture of a sample and a given reagent that reacts to the sample;
a light irradiation means for irradiating the liquid mixture in the sample cell with light;
a light intensity detection means for receiving scattered light or transmitted light of the light radiated onto the liquid mixture by the light irradiation means so as to detect the intensity of the scattered light or transmitted light; and
the calculation means for calculating temporal changes in the intensity of the scattered light or transmitted light detected by the light intensity detection means, or in a value obtained by subjecting the intensity thereof to a given mathematical operation, **characterized in that**:
a timer that, when an operator sets a switch to ON, initiates a count for a time elapsed since a point in time when the switch has been set to ON;
a time broadcasting means for continuously broadcasting to the operator the time until an initiation time of a preparation period, in which a length of the preparation period is predetermined as the period for generating the liquid mixture by mixing the sample and reagent and then preparing the same for measurement, and the initiation time of the preparation period is taken as a time a given period of time has elapsed since the point in time when the switch has been set to ON;
a preparation period initiation broadcasting means for broadcasting to the operator the initiation of the preparation period, wherein
the presence or absence, or extent of progression, of the reaction between the sample and reagent is measured based on the temporal changes in the intensity of the scattered light or transmitted light, or in the value obtained by subjecting the intensity thereof to a given mathematical operation, which are calculated by the calculation means, after the end time of the preparation period.

2. The optical reaction measurement device according to claim 1, wherein
the time broadcasting means broadcasts to the operator the time until the initiation of the preparation period by means of a countdown display.

3. The optical reaction measurement device according to claim 1 or 2, wherein
it is possible to adjust the length of the preparation period as well as the given period from the point in time when the switch has been set to ON until the initiation time of the preparation period.

4. The optical reaction measurement device according to any one of claims 1 to 3, wherein
the retaining means is capable of retaining a plurality of sample cells,
it is possible to independently measure the presence or absence, or extent of progression, of the reaction between the sample and reagent for the liquid mixtures inside the plurality of sample cells,
the length of the preparation period as well as the given period from the point in time when the switch has been set to ON until the initiation time of the preparation period is set to the same value for the liquid mixtures in the plurality of sample cells, and
once the switch is set to ON, the time broadcasting means and the preparation period initiation broadcasting means operate for the liquid mixtures in the plurality of sample cells.

5. The optical reaction measurement device according to any one of claims 1 to 3, wherein
the sample is a sample targeting detection of, or measurement of the concentration of, endotoxin or β-D-glucan, and
the given reagent is LAL, which is a hemocyte extract of limuli.

6. The optical reaction measurement device according to claim 5, wherein
the time measurement value associated with the intensity of the scattered light or transmitted light as detected by the light intensity detection means, or with the value obtained by subjecting the intensity thereof to a given mathematical operation, is a time elapsed since the initiation time of the preparation period.

7. An optical reaction measurement method, comprising:
irradiating a liquid mixture of a sample and a given reagent that reacts to the sample with light;
detecting an intensity of scattered light or transmitted light of the light radiated onto the liquid mixture; and
measuring the presence or absence, or extent of progression, of a reaction between the sample and reagent based on temporal changes in the detected intensity of the scattered light or transmitted light, or in a value obtained by subjecting the intensity to a given mathematical operation, **characterized in that**:
an intention indicating step for indicating an intention to initiate measurement after a measurer mixes the sample and reagent to prepare the liquid mixture;
a time broadcasting step for continuously broadcasting to the measurer the time until the initiation of a preparation period, in which a length of the preparation period is predetermined as the period for generating the liquid mixture by mixing the sample and the reagent and then preparing the same for measurement, and the initiation time of the preparation period is taken as the time a given period of time has elapsed since the point in time when the intention indication has been performed; and
apreparationperiodinitiationbroadcastingstep for broadcasting to the measurer the initiation of the preparation period, wherein
the presence or absence, or extent of progression, of the reaction between the sample and reagent is measured based on changes in the intensity of the scattered light or transmitted light or in a value obtained by subjecting the intensity thereof to a given mathematical operation, after the end time of the preparation period.

8. The optical reaction measurement method according to claim 7, wherein
the presence or absence, or extent of progression, of the reaction between the sample and reagent is independently measured for a plurality of liquid mixtures,
the intention indication in the intention indicating step is the indication of an intention to prepare a plurality of liquid mixtures by mixing the sample and the reagent as well as to begin measuring the plurality of liquid mixtures,
the length of the preparation period and the given time from the point in time when the intention is indicated until the initiation time of the preparation period are set to the same value for the plurality of liquid mixtures, and
the time broadcasting step and the preparation period initiation broadcasting step are executed for the plurality of liquid mixtures in the sample cells when the intention is indicated.

9. The optical reaction measurement method according to claim 7 or 8, wherein
the sample is a sample targeting detection of, or measurement of the concentration of, endotoxin or β-D-glucan, and
the given reagent is LAL, which is a hemocyte extract of limuli.

10. The optical reaction measurement method according to claim 9, wherein
the time measurement value associated with the intensity of the scattered light or transmitted light, or with the value obtained by subjecting the intensity thereof to a given mathematical operation, is a time elapsed since the initiation time of the preparation period.
